(19) Europäisches Patentamt
European Patent Office
Office européen des brevets

(11) EP 0 553 629 B1

(12) EUROPÄISCHE PATENTSCHRIFT

(45) Veröffentlichungstag und Bekanntmachung des Hinweises auf die Patenterteilung:
**13.03.1996 Patentblatt 1996/11**

(51) Int Cl.[6]: **C07C 275/24**, C07C 309/46, C07C 309/15, C07C 309/47, C08F 2/24, C08F 12/14

(21) Anmeldenummer: **93100336.2**

(22) Anmeldetag: **12.01.1993**

(54) **Polymerisierbare Emulgatoren und Reaktivgruppen sowie Polymere aus Emulgatoren und anderen Monomeren**

Polymerisable emulsifiers and reactiv groups and polymers of emulsifiers and other monomers

Emulsifiants et groupes réactifs polymérisables et polymères d'émulsifiants et d'autres monomères

(84) Benannte Vertragsstaaten:
**BE DE FR GB**

(30) Priorität: **25.01.1992 DE 4202050**

(43) Veröffentlichungstag der Anmeldung:
**04.08.1993 Patentblatt 1993/31**

(73) Patentinhaber: **BAYER AG**
**D-51368 Leverkusen (DE)**

(72) Erfinder:
- **Heiliger, Ludger, Dr.**
  **W-5090 Leverkusen 1 (DE)**
- **Schmidt, Adolf, Dr.**
  **W-5000 Köln 80 (DE)**
- **Probst, Joachim, Dr.**
  **W-5090 Leverkusen 1 (DE)**

(56) Entgegenhaltungen:
**GB-A- 2 075 970**
**US-A- 4 429 096**
**US-A- 4 604 439**
**US-A- 4 962 154**

- **JOURNAL OF POLYMER SCIENCE, POLYMER SYMPOSIA Bd. 72, 1985, NEW YORK US Seiten 45 - 54 D. A. UPSON 'Reactive Functional Latex Polymers'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft polymerisierbare Sulfon- und Carbonsäurederivate, Verfahren zu deren Herstellung sowie ihre Verwendung als Emulgatoren für wäßrige Emulsionen, bei Carbonsäurederivaten auch als Reaktivgruppen zur Verknüpfung mit biologisch aktiven Substraten, wie Proteine und Nukleinsäuren. Die Erfindung betrifft weiterhin Polymere, welche aus den Emulgatoren und anderen Monomeren aufgebaut sind.

Polymerisierbare hydrophile Monomere und polymerisierbare oberflächenaktive Monomere sind bekannt (D. A. Upson, J. Polym. Sc., Polym. Symp. 72, 45 (1985)). Sie unterliegen jedoch Einschränkungen, die ihre Anwendbarkeit erheblich eingrenzen. Hydrophile, d.h. wasserlösliche Monomere sowie die bisherigen polymerisierbaren Emulgatoren, die in ihrem polymerisierbaren Teil entweder aus polaren (Meth-)Acryl-Gruppierungen oder aus olefinischen Doppelbindungen bestehen, zuzüglich einer weiteren, die Emulgatoreigenschaft bewirkenden, meist ionischen Gruppierung, besitzen die unerwünschte Eigenschaft, statt des erwünschten Einpolymerisierens in die Latexphase eine Polymerisation in der wäßrigen Phase als Nebenreaktion einzugehen (C. Pichot, Makromol. Chem., Macromol. Symp. 35/36, 327 (1990). Dies ist aus ökologischer und ökonomischer Sicht bedenklich, da eine polymere Verunreinigung des wäßrigen Serums zu Problemen bei der Entsorgung führen kann, und dieser im Serum vorhandene Teil prinzipiell einen für die eigentliche Reaktion (d.h. Aufbau eines Latex) nicht zugänglichen oder nur teilweise zugänglichen Reaktanten darstellt.

Dementsprechend können bisherige Emulgatoren auch nicht zu Verknüpfungsreaktionen mit biologisch aktiven Substanzen herangezogen werden, sondern es müssen zusätzliche einpolymerisierbare Reaktivgruppen der Polymerisationsmischung zugesetzt (US-P 4 962 154) oder in einem nachgeschalteten Schritt eingeführt werden. Dies erfordert einen zusätzlichen synthetischen Aufwand, insbesondere bezüglich der Zugänglichkeit der Reaktivgruppe für die Verknüpfungsreaktion mit der biologisch aktiven Substanz in wäßriger Phase, die nur an der Polymeroberfläche ablaufen kann.

Von unaktivierten unpolaren olefinischen Doppelbindungen ist bekannt, daß die Einpolymerisierbarkeit aufgrund der vorhandenen allylständigen Wasserstoffatome, die zu Übertragungsreaktionen neigen, sehr begrenzt ist. Im Extremfall führt dies dazu, daß nur ein Emulgatormolekül pro Polymerkette eingebaut wird (W. de Winter, A. Marien, Makrom. Chem. Rapid. Commun., 5, 593, (1984)). Dadurch werden die Molmassen der Polymeren in der Latexphase prinzipiell auf niedrige Werte begrenzt und/oder ein Teil des Emulgators wird nicht kovalent an den Latex gebunden, sondern lediglich physikalisch adsorbiert. Er befindet sich dann dem Verteilungsgleichgewicht entsprechend im wäßrigen Serum.

Erfindungsgemäße Verbindungen besitzen dagegen unpolare (alphamethyl-)styrol-funktionelle polymerisierbare Gruppen, die hervorragend mit den üblichen Latexmonomeren, wie beispielsweise (Alphamethyl-)Styrole und (Meth-)Acrylate copolymerisierbar sind. Die erfindungsgemäßen Verbindungen zeigen keine Tendenz zur Polymerisation in der wäßrigen Serumsphase. Dadurch ist die wäßrige Phase fast völlig frei von Emulgatorresten, was ökologisch vorteilhaft ist. Zusätzlich werden durch die chemische Ankopplung dieser Emulgatoren die Latices elektrolyt- und scherstabiler sowie völlig reversibel reemulgierbar.

Darüber hinaus sind erfindungsgemäße Verbindungen, die eine Carbonsäuregruppierung besitzen, mit biologisch aktiven Substanzen, wie Proteinen oder amino-funktionalisierten Nucleinsäuren, beispielsweise über die bekannte N-Hydroxysuccinimid-Aktivierung, chemisch in wäßriger Phase verknüpfbar. Dies ermöglicht eine verbesserte Ankopplung dieser biologisch aktiven Substanzen an Latices oder generell an synthetische Polymere, die mit Hilfe von erfindungsgemäßen Verbindungen hergestellt werden. Zusätzliche Reaktivmonomere werden dabei nicht benötigt, da erfindungsgemäße Verbindungen aufgrund ihrer oberflächenaktiven Eigenschaften für die Kopplungsreaktion zugänglich sind. Dadurch können die biologisch aktiven Substanzen mit Polymeren markiert werden, was zu ihrer Immobilisierung und/oder für Nachweisreaktionen herangezogen werden kann.

Die erfindungsgemäßen Substanzen entsprechen der allgemeinen Formel (I)

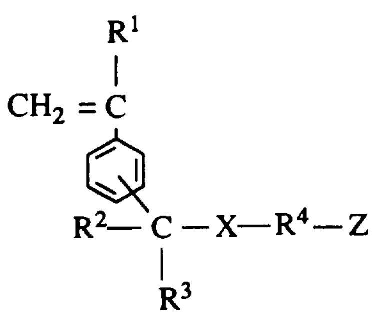

(I),

wobei

X NH-CO-Y oder Y,

| | |
|---|---|
| Y | NH, Sauerstoff oder Schwefel, |
| Z | COOM oder $SO_3M$, |
| M | Wasserstoff, Natrium, Ammonium oder Kalium, |
| $R^1$, $R^2$ und $R^3$ | unabhängig voneinander Wasserstoff oder Methyl und |
| $R^4$ | $C_1$-$C_{12}$-Alkylen, $C_3$-$C_{12}$-Cycloalkylen, Phenylen oder Naphthylen bedeuten. |

Bevorzugt sind Verbindungen der Formel (I), in denen X für NH-CO-NH oder NH-CO-O, $R^1$, $R^2$ und $R^3$ für Methyl und $R^4$ für $C_2$-$C_{11}$-Alkylen, insbesondere $C_5H_{10}$ oder $C_{10}H_{20}$, oder Phenylen stehen und der Rest

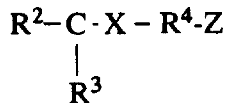

in meta-Position steht.

Besonders bevorzugt sind die Verbindungen der Formel (I), in denen X für

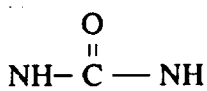

und Z für COOM und M für Natrium, Kalium oder Ammonium stehen.

Im einzelnen seien folgende Verbindungen genannt:

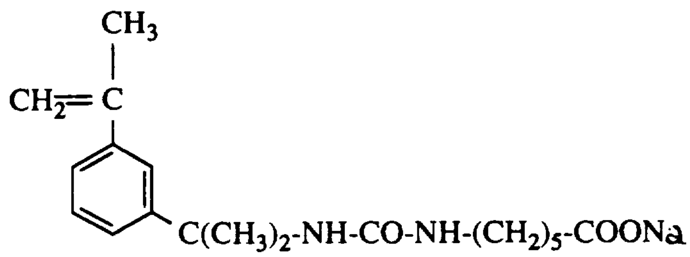

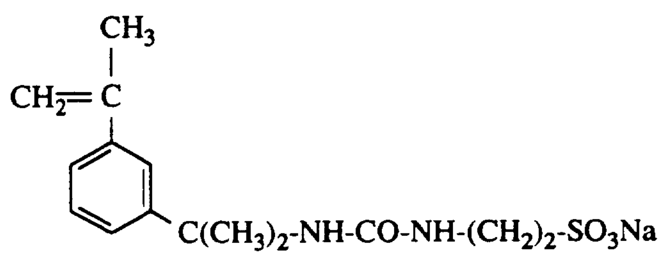

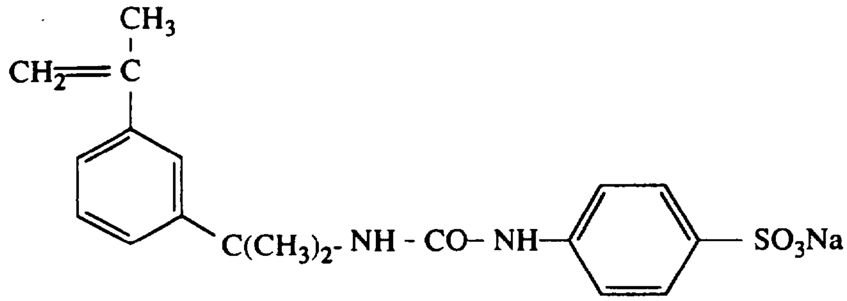

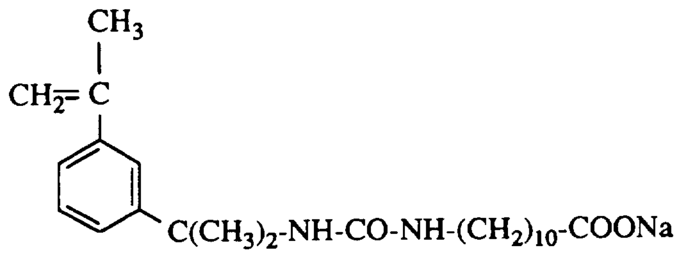

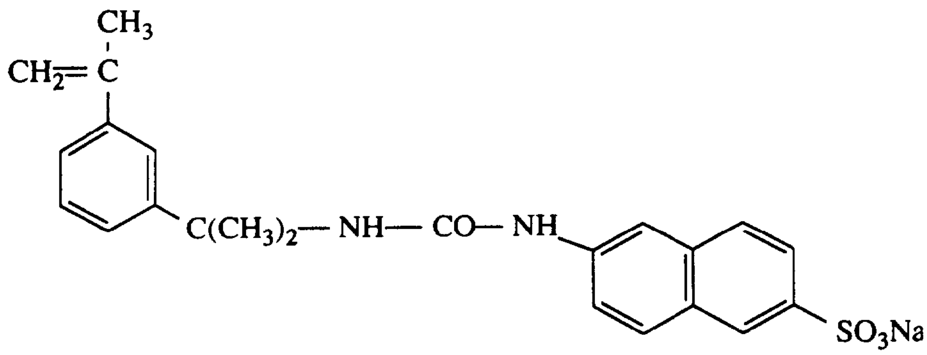

Es wurde weiterhin ein Verfahren zur Herstellung von erfindungsgemäßen Verbindungen gefunden, dadurch gekennzeichnet, daß man Styrolderivate der Formel (II)

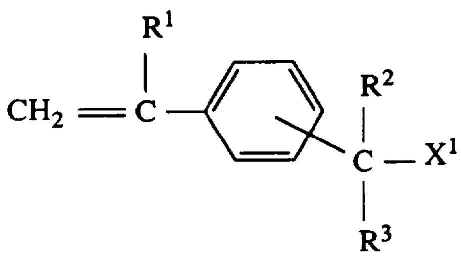

(II),

wobei

| | |
|---|---|
| $X^1$ | für NCO oder Chlor steht und |
| $R^1$, $R^2$ und $R^3$ | unabhängig voneinander für Wasserstoff oder Methyl stehen, mit Aminosulfon- oder Aminocarbonsäuren oder Hydroxylsulfon- oder Hydroxycarbonsäuren der Formel (III) |

$$X^2\text{-}R^4\text{-}Z, \qquad \text{(III)},$$

wobei

| | |
|---|---|
| $X^2$ | OH oder $NH_2$, |
| $R^4$ | $C_1$ bis $C_{12}$-Alkylen, $C_3$-$C_{12}$-Cycloalkylen, Phenylen oder Naphthylen und |
| Z | $SO_3M$ oder COOM bedeuten und |
| M | Waserstoff, Natrium, Kalium oder Ammonium bedeutet, umsetzt. |

Die Versuchsbedingungen zur Herstellung erfindungsgemäßer Verbindungen entsprechen denen von Additionen an Isocyanate, Etherbildungen sowie Alkylierungen von Aminen, die bekannt sind. Beispielsweise wird das Isocyanat mit dem zu reagierenden Amin oder Alkohol in einem inerten organischen Lösungsmitteln, beispielsweise aus der Gruppe der Ketone, Sulfoxide, Amide, Ether oder Halogenalkane, bei Temperaturen von 20-200°C umgesetzt.

Bei Verwendung von Aminogruppen enthaltenden Säuren muß die Aminogruppe mit einem Equivalent einer Hilfsbase wie beispielsweise Natriumhydroxid vor der Veresterung in ihre freie Form überführt werden. Es kann sich auch als vorteilhaft erweisen, die Veresterung in einer Zweiphasenreaktion (Wasser/Methylenchlorid) durchzuführen und einen Phasentransferkatalysator wie beispielsweise Benzyltrimethylammoniumhydroxid zuzusetzen.

Die erfindungsgemäßen Verbindungen können durch Verdampfung des Lösungsmittels in der Regel rein gewonnen werden oder auch in wäßriger Lösung direkt zur Emulgierung eingesetzt werden.

Die Erfindung betrifft weiterhin die Verwendung erfindungsgemäßer Verbindungen der allgemeinen Formel (I) als Emulgatoren für die wäßrige Emulsionspolymerisation. Erfindungsgemäße Verbindungen der Formel (I), in denen Z für COOM steht, können zusätzlich auch als Reaktivgruppen zur Anknüpfung biologisch aktiver Substrate an synthetische Polymere bzw. deren Latices verwendet werden.

Die Anknüpfung der biologisch aktiven Substrate an die Polymeren, die die Reaktivgruppen enthalten, kann über die Aktivierung der Carbonsäuregruppe mit N-Hydroxysuccinimid/Dicyclohexylcarbodiimid erfolgen. Das Polymer mit dem so aktivierten Ester kann beispielsweise über die primären Aminogruppen vieler biologisch aktiver Substanzen unter N-Hydroxysuccinimid - Abspaltung und Ausbildung einer Amidbildung mit biologisch aktiven Substraten verknüpft werden. Als synthetische Polymere seien beispielsweise Poly(meth-)acrylsäureester, Polyacrylnitril, Polyacrylamid, Polyamid oder Polystyrol und deren Derivate, bzw. deren Latices genannt. Enthält das Polymer beispielsweise ein oder mehrere Fluorophore, kann das biologisch aktive Molekül auf diese Weise fluoreszenzspektroskopisch nachgewiesen werden. Dieser Nachweis ist für medizinisch-diagnostische Anwendung wichtig.

Erfindungsgemäße Verbindungen der allgemeinen Formel (I) ermöglichen die Herstellung von völlig redispergierbaren Latices, d.h. mittels pH-Erniedrigung oder Ionenstärke-Erhöhung lassen sich die Latices völlig reversibel ausfällen und bei pH-Erhöhung oder Verdünnung wieder emulgieren ohne Veränderung der physikalischen Eigenschaften der Latices. Solche Emulsionen finden auf Grund ihrer verbesserten Stabilität gegenüber Scherkräften, Elektrolyten und Temperatureinflüssen (Auftau- und Gefriervorgänge) Verwendung als Wasserlacke (Bautenschutz, Industrielackierung), Leder- und Papierbeschichtungsmittel. Es ist möglich, mit erfindungsgemäßen Verbindungen Latices mit einem Feststoffgehalt von praktisch 100 % herzustellen.

Erfindungsgemäße Verbindungen eignen sich daher zur Herstellung von Polymeren aus technisch wichtigen Monomeren wie beispielsweise Styrol, Acrylnitril, (Meth)Acrylsäureester, (Meth)Acrylamide, Butadien, Vinylchlorid sowie Vinylacetat und deren Gemische. Polymere aus den erfindungsgemäßen Verbindungen der Formel (I) und weiteren Monomeren als Comonomere (vgl. beispielsweise die bereits oben und die im weiteren genannten Monomeren) sind ebenfalls Gegenstand der vorliegenden Erfindung.

Als fluoreszierende Comonomere kommen beispielsweise in Frage (Meth)Acrylate und/oder (Meth)Acrylamide, die über Ester- oder Amid-Verknüpfung Cumarine der Formel (IV) enthalten

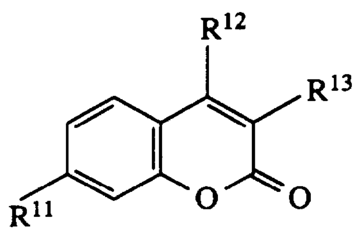

(IV),

in der

$R^{11}$ für O-Alkyl, $N(Alkyl)_2$, NH-Alkyl, $NH-SO_2$-Alkyl, O-Trimethylsilyl oder $NH-SO_2$-Aryl,

$R^{12}$ für Wasserstoff, Cyano, Chlor, Hydroxy, Alkyl oder Aryl und

$R^{13}$ für Phenyl oder Hetaryl stehen.

Alkyl bedeutet dabei vorzugsweise $C_1$- bis $C_6$-Alkyl, Aryl, vorzugsweise Phenyl, Alkylen, vorzugsweise $C_1$-$C_6$-Alkylen, und Hetaryl, vorzugsweise (Benz)thiazolyl,

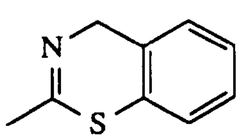

,

$R^{11}$ kann außerdem

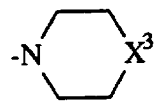

bedeuten, wobei

$X^3$ für Sauerstoff, N-$C_1$- bis $C_4$-Alkyl oder $(CH_2)_n$ steht, worin n = 0 oder 1 sein kann.

Weiterhin als Comonomere geeignet sind (Meth)Acrylate und/oder (Meth)Acrylamide, die ebenfalls über Ester- oder Amid-Verknüpfung Cumarine der Formel (V) enthalten,

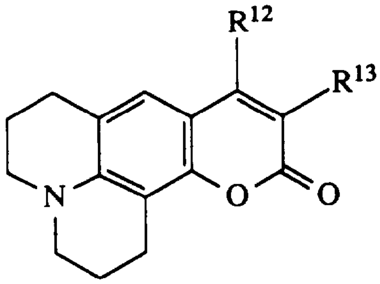

(V),

worin

$R^{12}$ und $R^{13}$ die bei Formel (IV) angegebene Bedeutung haben,

Vorzugsweise enthalten die Cumarine der Formeln (IV) und (V) an einem der Substituenten $R^{11}$, $R^{12}$ und $R^{13}$ eine funktionelle Gruppe für die Verknüpfung des Farbstoffs mit der (Meth)Acrylsäure bzw. deren reaktionsfähigen Derivaten oder dem daraus zugänglichen Polymer. $NH_2$- oder OH-Gruppen sind dafür besonders geeignet.

Ebenfalls als Comonomere geeignet sind (Meth)Acrylate und/oder (Meth)Acrylamide, die Carbostyrile der Formel (VI)

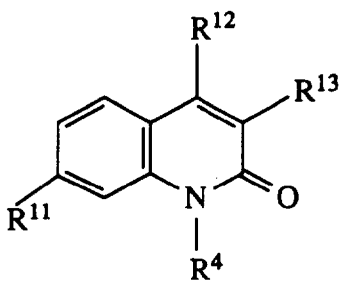

(VI),

worin

$R^{11}$, $R^{12}$ und $R^{13}$ die bei den Cumarinen (siehe Formeln (IV) und (V) angegebenen Bedeutungen haben und

$R^{14}$ für Alkyl, vorzugsweise für $C_1$- bis $C_6$-Alkyl steht.

Auch hier enthält einer der Substituenten vorzugsweise eine funktionelle Gruppe für die Verknüpfung mit der (Meth) Acrylsäure bzw. deren reaktionsfähigen Derivaten oder dem daraus zugänglichen Polymer.

Weiterhin als Comonomere sind (Meth)Acrylate und/oder (Meth)Acrylamide geeignet, die über Ester- oder Amid-Verknüpfung Pyrazoline der Formel (VII) enthalten,

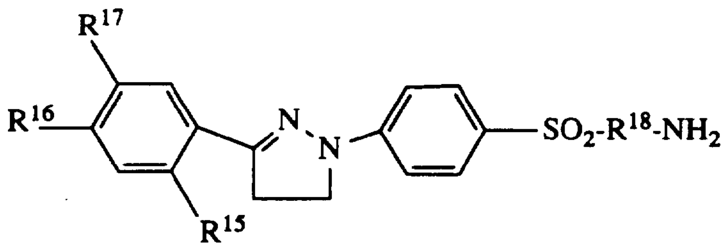

(VII),

worin

| | |
|---|---|
| $R^{15}$ | für Wasserstoff oder Methyl, |
| $R^{16}$ und $R^{17}$ | unabhängig voneinander für Wasserstoff oder Chlor und |
| $R^{18}$ | für Alkylen, |

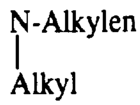

oder Alkylen-O-Alkylen stehen,
wobei Alkyl und Alkylen vorzugsweise $C_1$- bis $C_6$-Alkyl oder $C_1$- bis $C_6$-Alkylen bedeuten.

Auch geeignet sind Naphthalimide der Formel (VIII)

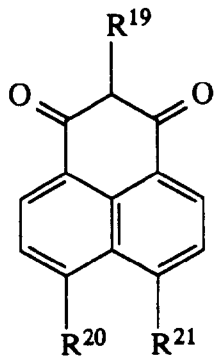

(VIII),

worin

| | |
|---|---|
| $R^{19}$ | für Alkyl und |
| $R^{20}$ und $R^{21}$ | unabhängig voneinander für Wasserstoff, O-Alkyl oder $N(Alkyl)_2$ stehen, wobei Alkyl vorzugsweise jeweils $C_1$- bis $C_6$-Alkyl bedeutet und einer der Reste $R^{19}$, $R^{20}$ oder $R^{21}$ eine $NH_2$-Gruppe zur Verknüpfung mit der (Meth)Acrylsäure bzw. deren reaktionsfähigen Derivaten oder dem daraus zugänglichen Polymer trägt. |

Ebenfalls geeignet sind Pyrene der Formel (IX)

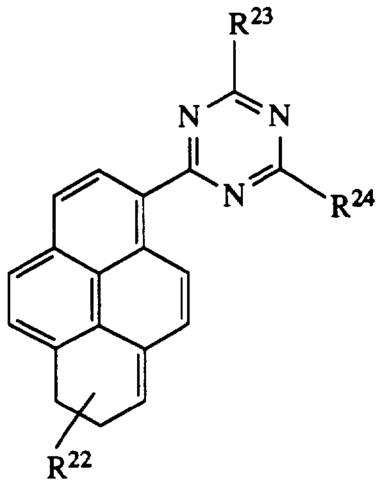

(IX),

worin

R²² für Wasserstoff oder $SO_3H$ und

$R^{23}$ und $R^{24}$ unabhängig voneinander für O-Alkyl oder $N(Alkyl)_2$ stehen, wobei Alkyl vorzugsweise $C_1$- bis $C_6$-Alkyl bedeutet und einer der Reste $R^{23}$ oder $R^{24}$ eine $NH_2$-Gruppe zur Verknüpfung mit der (Meth)Acrylsäure bzw. deren reaktionsfähigen Derivaten oder dem daraus zugänglichen Polymer trägt.

Es kommen auch Fluoresceine der Formel (X)

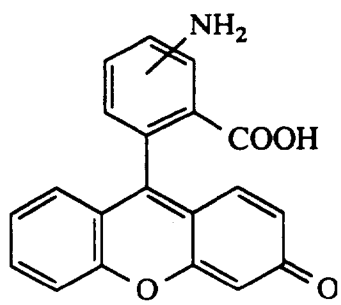

(X),

und Rhodanine der Formel (XI) in Frage

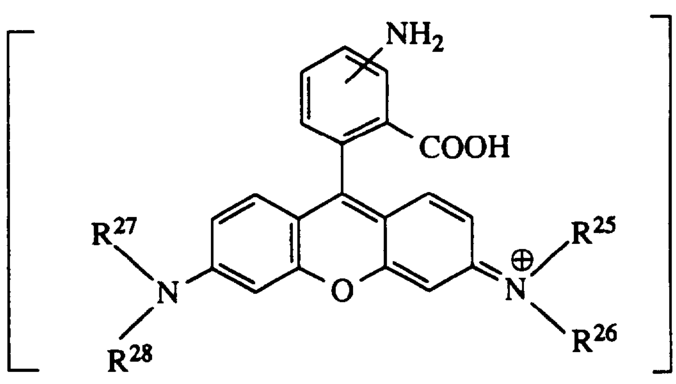

$Y^{\ominus}$ (XI),

worin

$Y^{\ominus}$ ein farbloses Anion, z.B. $Cl^{\ominus}$,$Br^{\ominus}$, $I^{\ominus}$, $HSO_4^{\ominus}$,

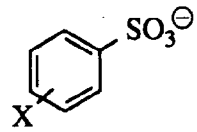

$X = Cl, Br, I, CH_3$
bedeutet und

$R^{25}$ bis $R^{28}$ unabhängig voneiner für Alkyl oder

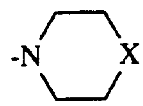

stehen, wobei Alkyl vorzugsweise $C_1$- bis $C_6$-Alkyl bedeutet und X für Sauerstoff, N-$C_1$- bis $C_4$-Alkyl oder $(CH_2)_n$ steht, worin n = 0 oder 1 sein kann.

$\overset{\oplus}{N}R^{25}R^{26}$ **und/oder** $NR^{27}R^{28}$

können auch zusammen mit dem aromatischen Ring, an den sie gebunden sind, ein polycyclisches System bilden, z.B. ein System der Formel (XII) oder (XIII).

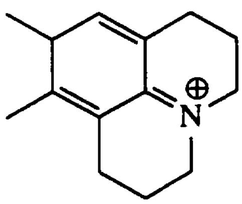
**(XII)**

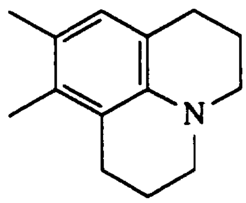
**(XIII).**

Diese und weitere geeignete Farbstoffe sind bekannt (siehe z.B. "The Chemistry of Synthetic Dyes", Vol. V, Academic Press (1971) und "Fluorescent Whitening Agents", G. Thieme Verlag Stuttgart (1975)).

Die in diesem Zusammenhang genannten Verbindungen enthalten an einem der Substituenten eine funktionelle Gruppe für die Verknüpfung mit der (Meth)Acrylsäure bzw. deren reaktionsfähigen Derivaten oder mit dem daraus zugänglichen Polymer. $NH_2$- oder OH-Gruppen sind dafür besonders geeignet.

Der Anteil der polymersisierbaren Emulgatoren der Formel (1) in den obengenannten Polymeren beträgt im allgemeinen 0,1 bis 50 Gew.-%, bevorzugt 1 bis 25 Gew.-% und besonders bevorzugt 2,5 bis 10 Gew.-%.

Die Polymere können linear, verzweigt oder vernetzt sein und lassen sich durch radikalische Polymerisation herstellen.

Nichtvernetzte Polymere haben mittlere Molekulargewichte ($\bar{M}_w$) zwischen 1 000 und 10 000 000, bevorzugt zwischen 5 000 und 2 000 000.

Die Polymere können in organischen Lösungsmitteln wie beispielsweise Aromaten, Dimethylsulfoxid, Dimethylformamid, Ester oder Ketonen hergestellt, dann wäßrig umgelöst oder auch direkt in Wasser als Latex hergestellt werden.

Die Erfindung wird durch folgende Beispiele näher erläutert.

**A) Synthese von Emulgatoren aus:**

Beispiele 1 -4:

1: TMI + $H_2N$-$(CH_2)_5$-COONa (TMI = m-Isopropenyl-alpha,alpha-dimethylbenzylisocyanat)

2: TMI + $H_2N$-$(CH_2)_{10}$-COONa

3: TMI + $H_2N$-$C_2H_4$-$SO_3Na$

4:

TMI + 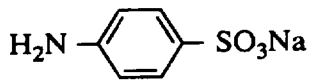

In eine Lösung aus 0,1 mol der obigen Amino-carbon bzw. sulfonsäuren und 0,1 mol Natriumhydroxid in 100 ml entionisiertem Wasser werden unter Stickstoff 100 ml Aceton zugegeben und 5 min lang gerührt. Dann wird tropfenweise 0,1 mol m-Isopropenyl-alpha,alpha-dimethylbenzylisocyanat in 50 ml Aceton gelöst, zudosiert, noch 1 h nachgerührt und das Aceton abdestilliert.

Für IR- und NMR-Spektroskopie werden Teile der Lösung bis zur Trockne eingedampft. Bei diesen Rückständen ist die Isocyanatbande im IR-Spektrum bei 2 200 $cm^{-1}$ durch eine Harnstoffbande bei 1 680 $cm^{-1}$ ersetzt. Im $^1H$-NMR-Spektrum befinden sich die charakteristischen Harnstoffprotonenresonanzen bei 6,3 bzw. 6,7 ppm.

**B) <u>Latexpolymerisationen</u>**

Beispiele 5 - 12:

Unter Rühren werden in 187,34 bzw. 177,08 ml entionisiertem Wasser 8,94 g einer 1 gew.-%igen wäßrigen Natriumcarbonatlösung und jeweils 22,8 bzw. 34,2 g einer 10 gew.-%igen wäßrigen Emulgatorlösung aus Beispiel 1 - 4 eingetragen. Dann wird 57 g Styrol und 11,4 g einer 1 gew.-%igen wäßrigen Kaliumpersulfatlösung zugegeben, auf 70°C erhitzt und 19 h polymerisiert. Nach Erkalten wird der Rohlatex durch ein Polyamidnetz der Maschenweite 100 µ filtriert und das Filtrat auf Feststoffgehalt und mittlere Teilchengrößendurchmesser untersucht (Tabelle 1).

Die Latices gemäß Beispiele 5 bis 12 haben einen Feststoffgehalt von praktisch 100 % der Theorie.

Tabelle 1

Latices aus Styrol und Emulgatoren gemäß Beispiel 1 bis 4

| Latex Beispiel | Emulgator aus Beispiel | Gehalt an Emulgator (g Feststoff) | mittlere Teilchengrößen-Durchmesser in nm (photometrisch bei 700 nm) |
|---|---|---|---|
| 5 | 1 | 2,28 | 125 |
| 6 | 1 | 3,42 | 105 |
| 7 | 2 | 2,28 | 144 |
| 8 | 2 | 3,42 | 130 |
| 9 | 3 | 2,28 | 194 |
| 10 | 3 | 3,42 | 207 |
| 11 | 4 | 2,28 | 59 |
| 12 | 4 | 3,42 | 55 |

Redispergierungsversuche:

Je 10 ml der Latices gemäß den Beispielen 5 bis 12 werden bei Raumtemperatur im Hochvakuum getrocknet und durch Zugabe von 10 ml Wasser redispergiert. Die redispergierten Latices zeigen keinerlei Koagulatbildung und weisen gleiche mittlere Teilchengrößendurchmesser auf, wie die jeweiligen Ausgangslatices.

Serumsuntersuchungen:

Das nach Ultrazentrifugation untersuchte Serum enthielt keine löslichen Anteile und zeigte nach dem Eindampfen im Hochvakuum keine Spuren organischer Verbindungen ($^1$H-NMR, Dünnschichtchromatographie)

**C) Verbindungen der Formel (I) mit Z = COOM als Reaktivgruppe**

Beispiel 13

Herstellung von Markierungsreagenz mit Emulgator 1 als Verknüpfungsgruppe für biologisch aktive Moleküle
1 g Farbstoff der Formel

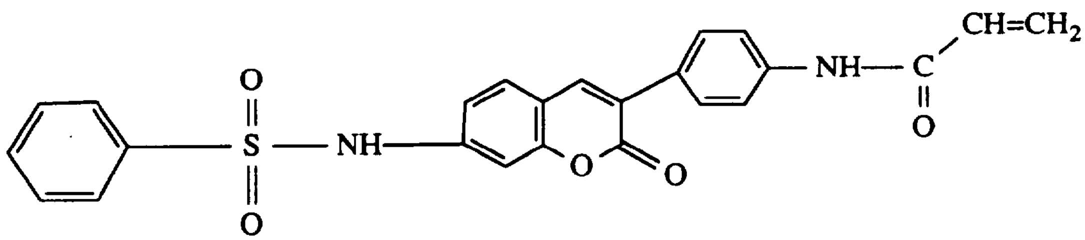

und 200 mg Emulgator aus Beispiel 1, 2,8 g Natrium-p-styrylsulfonat (Na-PSS) sowie 12 mg Azoisobuttersäuredinitril (AIBN) werden in 25 ml Dimethylsulfoxid eingetragen, die Apparatur evakuiert, mit Stickstoff begast, der Vorgang 3 mal wiederholt, die Lösung auf 65°C erhitzt und 15 h reagiert. Nach Erkalten wird die Lösung mit 150 mg Bis-(N-Succinimidyl) carbonat versetzt und 6 h bei Raumtemperatur gerührt. Die Reaktionslösung wird in 200 ml Methanol eingetropft, der entstandene Niederschlag abfiltriert und getrocknet. Das Rohpolymer wird einer Ultrafiltration (Cutoff 10.000 Dalton) unterworfen.
Ausbeute 80 %

Beispiel 14

Verwendung von Markierungsreagenz aus Beispiel 13 in Immunologischen Testsystemen

10 mg Polymer gemäß Beispiel 13 werden in 10 ml Carbonat-Bicarbonatpuffer pH 9,0/0,5 molar gelöst. Dazu werden 5 mg Phosphordiesterase (PDE; aus Klapperschlangengift, Firma Sigma) in 5 ml Carbonatpuffer pH 9,0 (0,5 M) zugegeben, 4 h bei Raumtemperatur gerührt, ber Nacht im Kühlschrank weiterreagieren lassen. Danach wird mit 1n NaOH auf pH 11 gestellt und die Rohlösung chromatographiert (Seplacryl S-500 der Firma Pharmacia in Carbonatpuffer 0,02 m, pH 11, Ø der Säule 16 mm, Höhe 100 cm).

Nach Durchlauf von 500 ml des Carbonatpuffers wurde mit ca. 1 l einer 25%igen Ammoniaklösung 1:10 in Wasser gewaschen.

Der erste Peak bei 200 ml Elutionsvolumen enthält PDE-Aktivität* und Fluoreszenz.

Nicht umgesetzter Farbstoff und nicht umgesetztes PDE eluieren später.

* Prüfung auf PDE-Aktivität:

| | |
|---|---|
| 400 µl | Eluat |
| 600 µl | Trispuffer pH 8,8/0,1 M |
| 200 µl | Mg-acetat 0,3 M und |
| 1000 µl | Bis-p-nitrophenylphosphat 1 mmolar |
| | werden 105 min bei 37°C reagiert, wobei Gelbfärbung sichtbar wird. |

Beispiel 15

Verwendung von Markierungsreagenz aus Beispiel 13 für Gensonden

Es werden 100 µg des Aminolinkoligonukleotids mit der Sequenz CTC GGA TCC CAT CTT CTC CCC TGA GTC TGT (Synthese gemäß N. D. Sinha u. R. M. Cook, Nucleic Acids Research 16, 2659 (1988) in 300 µl Carbonatpuffer (pH = 9) gelöst und mit einem Überschuß an polymerem Fluoreszenzfarbstoff gemäß Beispiel 13 in 200 µl Carbonatpuffer versetzt. Die Reaktion erfolgt über 60 h bei Raumtemperatur. Die Aufarbeitung erfolgt durch Gelfiltration an Biorad-Bio-Gel P 4 oder durch reversed phase HPLC an RP 18-Säule mit Triethylammoniumacetat/Acetonitril als Eluenten.

Beispiel 16

100 µg des Aminolinkoligonukleotids der Sequenz AT CTA CTG GCT CTT TTT TTT TTT TTT TTT TTT TTT TTT TTT T werden in 200 µl Carbonatpuffer gelöst (pH = 9) und mit einem Überschuß polymerem Fluoreszenzfarbstoff gemäß Beispiel 13 in 300 µl Carbonatpuffer versetzt und für 60 h bei Raumtemperatur gerührt. Zur Aufarbeitung wird das gesamte Reaktionsgemisch auf eine Poly A-Sepharose 4B-Säule (5 ml) (Pharmacia) aufgetragen. Mit dem Auftragepuffer (A) (s. u., 20 ml) wird der nichtgebundene Farbstoff heruntergewaschen, dann wird mit dem Elutionspuffer (B) (s. u., 30 ml) gewaschen und schließlich das Kopplungsprodukt mit Elutionspuffer (C) (s. u., 40 ml) heruntergewaschen.

Auftragepuffer (A): 1,513 g Tris, 10,23 g NaCl, 0,56 g EDTA in 750 ml Wasser lösen und mit Formamid auf 1 Liter auffüllen.

Elutionspuffer (B): In 100 ml Auftragepuffer A 7,45 g KCl lösen.

Elutionspuffer (C): 3,7 g KCl und 50 ml Formamid mit Auftragepuffer A auf 100 ml auffüllen.

Der so markierte Aminolink-Oligonukleotid kann in DNA-Probe-Tests direkt zum Nachweis einer komplementären Nukleotidsequenz eingesetzt werden.

**Patentansprüche**

1. Verbindungen der allgemeinen Formel (I)

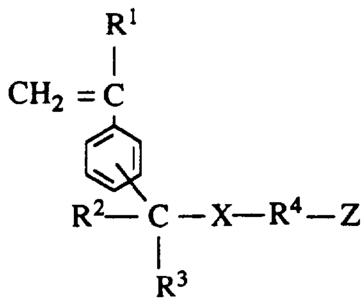

(I),

wobei

| | |
|---|---|
| X | NH-CO-Y oder Y, |
| Y | NH, Sauerstoff oder Schwefel, |
| Z | COOM oder $SO_3M$, |
| M | Wasserstoff, Natrium, Ammonium oder Kalium, |
| $R^1$, $R^2$ und $R^3$ | unabhängig voneinander Wasserstoff oder Methyl und |
| $R^4$ | $C_1$-$C_{12}$-Alkylen, $C_3$-$C_{12}$-Cycloalkylen, Phenylen oder Naphthylen bedeuten. |

2. Verbindungen der Formel (I) gemäß Anspruch 1, wobei X für NH-CO-NH oder NH-CO-O, $R^1$, $R^2$ und $R^3$ für Methyl und $R^4$ für $C_2$-$C_{11}$-Alkylen oder Phenylen stehen und der Rest

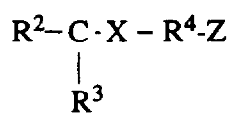

in meta-Position steht.

3. Verbindungen der Formel (I) gemäß Anspruch 1 und 2, wobei Z für COOM und M für Natrium, Kalium oder Ammonium stehen.

4. Verfahren zur Herstellung von Verbindungen der Formel (I)

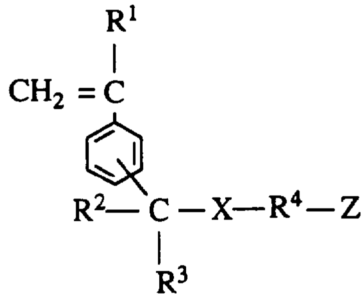

(I),

wobei

| | |
|---|---|
| X | NH-CO-Y oder Y, |
| Y | NH, Sauerstoff oder Schwefel, |
| Z | COOM oder $SO_3M$, |
| M | Wasserstoff, Natrium, Ammonium oder Kalium, |
| $R^1$, $R^2$ und $R^3$ | unabhängig voneinander Wasserstoff oder Methyl und |
| $R^4$ | $C_1$-$C_{12}$-Alkylen, $C_3$-$C_{12}$-Cycloalkylen, Phenylen oder Naphthylen bedeuten, wobei man Styrolderivate der Formel (II), |

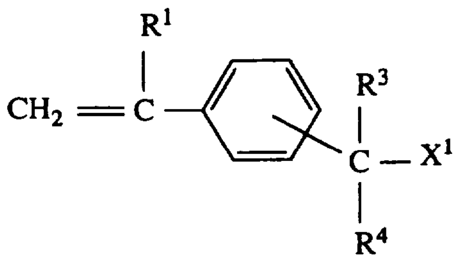

(II),

in welcher

| | |
|---|---|
| $X^1$ | für NCO oder Chlor steht und |
| $R^1$, $R^2$ und $R^3$ | unabhängig voneinander für Wasserstoff oder Methyl stehen, mit Aminosulfon- oder Aminocarbonsäuren oder Hydroxylsulfon- oder Hydroxycarbonsäuren der Formel (III) |

$$X^2\text{-}R^4\text{-}Z, \qquad \text{(III)},$$

wobei

| | |
|---|---|
| $X^2$ | OH oder $NH_2$, |
| $R^4$ | $C_1$ bis $C_{12}$-Alkylen, $C_3$-$C_{12}$-Cycloalkylen, Phenylen oder Naphthylen und |
| Z | $SO_3M$ oder COOM bedeuten und |
| M | Waserstoff, Natrium, Kalium oder Ammonium bedeutet, umsetzt. |

**5.** Verwendung von Verbindungen der Formel (I) gemäß Anspruch 1 als Emulgatoren für die wäßrige Emulsionspolymerisation.

6. Verwendung von Verbindungen der Formel (I) gemäß Anspruch 1, in welchen Z für COOM steht, als Reaktivgruppen zur Anknüpfung biologisch aktiver Substrate an synthetische Polymere.

7. Polymere auf der Basis von mindestens einer Verbindung der Formel (I) gemäß Anspruch 1 und mindestens einem weiteren Monomer.

8. Polymere gemäß Anspruch 7, wobei das weitere Monomere ausgewählt ist aus Styrol, Arylnitril, (Meth)Acrylsäureester, (Meth)Acrylamide, Butadien, Vinylchlorid und Vinylacetat.

9. Polymere gemäß Anspruch 7, wobei der Anteil an Verbindungen der Formel (I) 0,1 bis 50 Gew.-% beträgt.

**Claims**

1. Compounds corresponding to general formula (I):

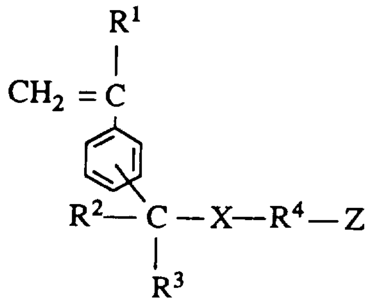

(I),

in which

| | |
|---|---|
| X | is NH-CO-Y or Y, |
| Y | is NH, oxygen or sulfur, |
| Z | is COOM or $SO_3M$, |
| M | is hydrogen, sodium, ammonium or potassium, |
| $R^1$, $R^2$ and $R^3$ | independently of one another represent hydrogen or methyl and |
| $R^4$ | is $C_{1-12}$ alkylene, $C_{3-12}$ cycloalkylene, phenylene or naphthylene. |

2. Compounds corresponding to formula (I) as claimed in claim 1, in which X represents NH-CO-NH or NH-CO-O; $R^1$, $R^2$ and $R^3$ represent methyl and $R^4$ represents $C_{2-11}$ alkylene or phenylene and the residue

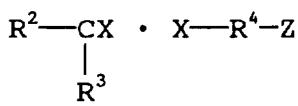

is in the meta position.

3. Compounds corresponding to formula (I) as claimed in claims 1 and 2, in which Z represents COOM and M stands for sodium, potassium or ammonium.

4. A process for the production of compounds corresponding formula (I):

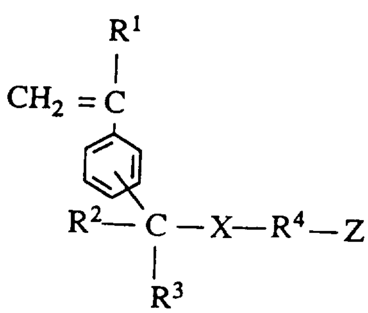

(I),

in which

| | |
|---|---|
| X | is NH-CO-Y or Y, |
| Y | is NH, oxygen or sulfur, |
| Z | is COOM or $SO_3M$, |
| M | is hydrogen, sodium, ammonium or potassium, |
| $R^1$, $R^2$ and $R^3$ | independently of one another represent hydrogen or methyl and |
| $R^4$ | is $C_{1-12}$ alkylene, $C_{3-12}$ cycloalkylene, phenylene or naphthylene, |

characterized in that styrene derivatives corresponding to formula (II):

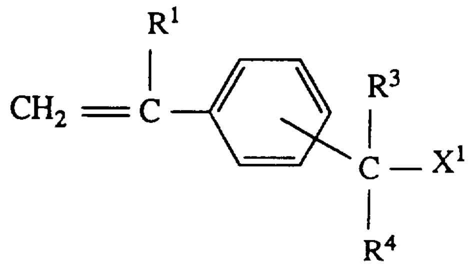

(II),

in which

| | |
|---|---|
| $X^1$ | is NCO or chlorine and |
| $R^1$, $R^2$ and $R^3$ | independently of one another represent hydrogen or methyl, |

are reacted with aminosulfonic or aminocarboxylic acids or hydroxysulfonic or hydroxycarboxylic acids corresponding to formula (III):

$$X^2\text{-}R^4\text{-}Z \qquad \text{(III)}$$

in which

| | |
|---|---|
| $X^2$ | is OH or $NH_2$, |
| $R^4$ | is $C_{1-12}$ alkylene, $C_{3-12}$ cycloalkylene, phenylene or naphthylene and |
| Z | represents $SO_3M$ or COOM and |
| M | is hydrogen, sodium, potassium or ammonium. |

5. The use of the compounds corresponding to formula (I) claimed in claim 1 as emulsifiers for aqueous emulsion polymerization.

6. The use of the compounds corresponding to formula (I) claimed in claim 1, in which Z is COOM, as reactive groups for linking biologically active substrates to synthetic polymers.

7. Polymers based on at least one compound corresponding to formula (I) as claimed in claim 1 and at least one other monomer.

8. Polymers as claimed in claim 7 in which the other monomer is selected from styrene, acrylonitrile, (meth)acrylates, (meth)acrylamides, butadiene, vinyl chloride and vinyl acetate.

9. Polymers as claimed in claim 7, in which the percentage content of compounds corresponding to formula (I) is 0.1 to 50% by weight.

## Revendications

1. Composés répondant à la formule générale (I)

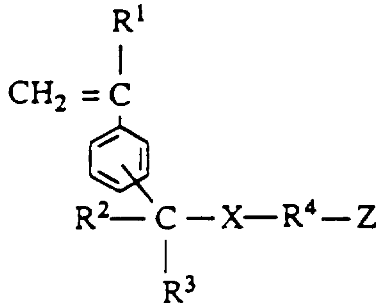

(I),

dans laquelle

| | |
|---|---|
| X | représente NH-CO-Y ou Y, |
| Y | représente NH, un atome d'oxygène ou un atome de soufre, |
| Z | représente COOM ou $SO_3M$, |
| M | représente un atome d'hydrogène, un atome de sodium, un groupe ammonium ou un atome de potassium, |
| $R^1$, $R^2$ et $R^3$ | représentent, indépendamment l'un de l'autre, un atome d'hydrogène ou un groupe méthyle, et |
| $R^4$ | représente un groupe alkylène en $C_1$-$C_{12}$, un groupe cycloalkylène en $C_3$-$C_{12}$, un groupe phénylène ou un groupe naphtylène. |

2. Composés de formule (I) selon la revendication 1, dans laquelle X représente NH-CO-NH ou NH-CO-O, $R^1$, $R^2$ et $R^3$ représentent un groupe méthyle et $R^4$ représente un groupe alkylène en $C_2$-$C_{11}$ ou encore un groupe phénylène, et le radical

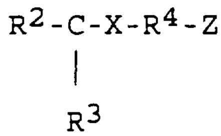

se trouve en position méta.

3. Composés de formule (I) selon les revendications 1 et 2, dans laquelle Z représente COOM et M représente un atome de sodium, un atome de potassium ou un groupe ammonium.

4. Procédé pour la préparation de composés de formule (I)

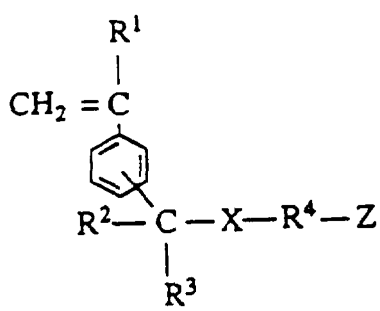

(I),

dans laquelle

| | |
|---|---|
| X | représente NH-CO-Y ou Y, |
| Y | représente NH, un atome d'oxygène ou un atome de soufre, |

Z représente COOM ou $SO_3M$,

M représente un atome d'hydrogène, un atome de sodium, un groupe ammonium ou un atome de potassium,

$R^1$, $R^2$ et $R^3$ représentent, indépendamment l'un de l'autre, un atome d'hydrogène ou un groupe méthyle, et

$R^4$ représente un groupe alkylène en $C_1$-$C_{12}$, un groupe cycloalkylène en $C_3$-$C_{12}$, un groupe phénylène ou un groupe naphtylène,

dans lequel on fait réagir des dérivés de styrène répondant à la formule (II)

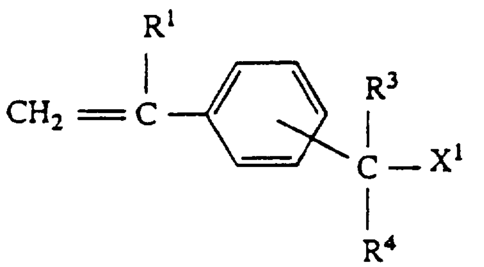

(II),

dans laquelle

$X^1$ représente NCO ou un atome de chlore, et

$R^1$, $R^2$ et $R^3$ représentent, indépendamment l'un de l'autre, un atome d'hydrogène, un groupe méthyle, avec des acides aminosulfoniques ou aminocarboxyliques, ou encore avec des acides hydroxysulfoniques ou hydroxycarboxyliques de formule (III)

$$X^2\text{-}R^4\text{-}Z \quad \text{(III)}$$

dans laquelle

$X^2$ représente OH ou $NH_2$,

$R^4$ représente un groupe alkylène en $C_1$-$C_{12}$, un groupe cycloalkylène en $C_3$-$C_{12}$, un groupe phénylène ou un groupe naphtylène, et

Z représente $SO_3M$ ou COOM, et

M représente un atome d'hydrogène, un atome de sodium, un atome de potassium ou un groupe ammonium.

**5.** Utilisation de composés de formule (I) selon la revendication 1, comme émulsifiants pour la polymérisation en émulsion aqueuse.

**6.** Utilisation de composés de formule (I) selon la revendication 1, dans lesquels Z représente COOM, comme groupes réactifs pour la liaison de substrats biologiquement actifs à des polymères synthétiques.

**7.** Polymères à base d'au moins un composé de formule (I) selon la revendication 1 et d'au moins un autre monomère.

**8.** Polymères selon la revendication 7, dans lesquels l'autre monomère est choisi parmi le groupe comprenant le styrène, l'acrylonitrile, des esters d'acide (méth)acrylique, des (méth)acrylamides, le butadiène, le chlorure de vinyle et l'acétate de vinyle.

**9.** Polymères selon la revendication 7, dans lesquels la fraction des composés de formule (I) s'élève de 0,1 à 50% en poids.